# EUROPEAN PATENT APPLICATION

(11) **EP 1 685 838 A1**
(43) Date of publication of application: **02.08.2006**
(21) Application number: 06007780.7
(22) Date of filing: 13.08.2001
(51) Int. Cl.: A61K 31/48, A61P 25/30, A61P 25/34, A61P 25/32

(54) **Compounds for the treatment of addictive disorders**

(30) Priority: 16.08.2000 US 225714 P; 23.01.2001 US 263610 P
(62) Divisional of application: 01962196.0
(71) Applicant: Pharmacia & Upjohn Company LLC, Kalamazoo, MI 49001 (US)
(72) Inventor: Anderson, Richard W., Annadale, NJ 08801 (US); McBrinn, Sylvia S., Stockton, NJ 08559 (US); Robertson, David W., Ann Arbor, MI 48113-1159 (US); Marshall, Robert C., Mattawan, MI 49071 (US)
(74) Representative: Perry, Robert Edward

(57) **Abstract**

Use of a compound for the preparation of a medicament for treating or suppressing the symptoms of at least one disorder selected from addictive disorders, psychoactive substance use disorders, intoxication disorders, inhalation disorders, alcohol addiction, tobacco addiction, and nicotine addiction, wherein the compound is of the formula: 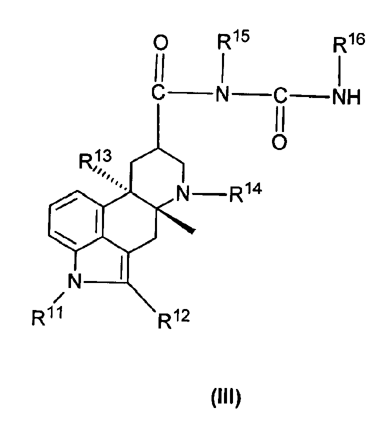 or a pharmaceutically acceptable salt thereof, wherein:
R¹¹ is hydrogen or methyl;
R¹² is hydrogen, halogen, methyl, formyl, S-R¹⁷, or SO-R¹⁷ wherein R¹⁷ is C₁-C₄ alkyl or phenyl;
R¹³ is hydrogen or methoxy;
R¹⁴ is C₁-C₄ alkyl, C₁-C₄ alkenyl, C₁-C₄ alkynyl, benzyl or phenyl; and
R¹⁵ and R¹⁶ are each independently C₁-C₄ alkyl, cyclohexyl, benzyl, phenyl optionally substituted with halogen or methoxy, or (CH₂)ₙN(CH₃)₂, wherein n is an integer.

## Description

### BACKGROUND OF THE INVENTION

### Field of the invention

The invention relates to the use of neuromuscular agents, and the pharmacologically acceptable salts thereof, for the treatment of, or improving symptoms of, several nervous system disorders. More particularly, the invention relates to treatment and improvement of symptoms related to addictive disorders, psychoactive substance use disorders, nicotine addiction, and tobacco addiction.

### Description of Related Technology

Several classes of compounds have been described for the effective treatment and management of the diseases fibromyalgia (FMS) (or fibromyalgia syndrome) and Chronic Fatigue Immune Disorders Syndrome (CFIDS) or Chronic Fatigue Syndrome (CFS). More particularly, heterocyclic amine type compounds, phenylazacycloalkane type compounds, cabergoline and cabergoline-type compounds have been described for the effective treatment and management of these neuromuscular conditions.

Heterocyclic amine compounds and methods of making the same are disclosed in U.S. Patent Nos. 5,273,975, issued December 28, 1993; U.S. 5,436,240, issued July 25, 1995; U.S. 5,462,947, issued October 31, 1995; and U.S. 5,594,024, issued January 14, 1997. More particularly, the compounds and the processes for making those compounds, formulations and methods of preparing medicaments are described in U.S. Patent No. 5,273,975, issued December 28, 1993; and U.S. Patent No. 5,436,240, issued July 25, 1995, also providing a generic description of compounds having use in the treatment of FMS and CFIDS.

Phenylazacycloalkane compounds and methods of making the same have been described in U.S. Patent Nos. 5,594,024, issued January 14, 1997, and U.S. 5,462,947, issued October 31, 1995. The compounds are disclosed as having useful activity in treating central nervous disorders related to dopamine receptor activity.

Cabergoline and cabergoline-type compounds have been disclosed as demonstrating hypotensive and antiprolactinic activity. The compound is commercially available from Pharmacia & UpJohn, Inc. (now Pharmacia Corporation) under the trade names DOSTINEX™ and CABASER™ for hyperprolactinemic disorders and Parkinson's disease. The compounds and methods for making the same are described in U.S. Patent No. 4,526,892, issued July 2, 1985.

More recently, scientists have considered whether these compounds having useful properties for treating neuromuscular disorders can be used for treating other nervous system disorders, particularly addictive diseases. More particularly, the use of these compounds for nervous systems disorders, for example, addictive disorders, psychoactive substance use disorders, nicotine addiction, or tobacco addiction resulting in smoking cessation, have been considered.

In addition to the previously mentioned compounds, aromatic bicyclic amine compounds have also been investigated for potential activity useful for treating nervous system disorders, such as addictive diseases. The aromatic bicyclic amine compounds have been reported to demonstrate activity useful for treatment of some central nervous system disorders, for example, schizophrenia, and cardiovascular disease, such as cardiac arrhythmias and cardiac fibrillation. Bicyclic amine compounds and methods of making the same are described in U.S. Patent No. 5,877,317, issued March 2, 1999.

Methods for using the described compounds for treating addictive-type nervous disorders has not been reported. Methods and dosages for using heterocyclic amine compounds, phenylazacycloalkane compounds, cabergoline, aromatic bicyclic amine compounds and the derivatives of these classes of compounds for treating specific addictive disorders are described herein.

### SUMMARY OF THE INVENTION

The invention provides a method for the treatment of certain addictive disorders, for example, psychoactive substance use disorders, nicotine addiction or tobacco addiction (with a result of smoking cessation or a decrease in smoking). The method includes the step of administering a therapeutically effective, nontoxic dose of a heterocyclic amine, a phenylazacycloalkane, a cabergoline, or an aromatic bicyclic amine compound, or a pharmaceutically acceptable salt or derivative thereof, to a patient suffering from or susceptible to such an addiction or disorder.

### DETAILED DESCRIPTION OF THE INVENTION

Heterocyclic amine, phenylazacycloalkane, cabergoline, aromatic bicyclic amine compounds, and the pharmaceutically acceptable salts or derivatives of these compounds can be used to treat and ameliorate nervous system disorders. The disorders typically can include, but are not limited to, addictive disorders, psychoactive substance use disorders, nicotine addition, tobacco addiction, and other diseases or disorders related to affliction of the nervous system, and more particularly, the central nervous system.

Several compounds demonstrating activity in treating neuromuscular disease have been identified for the method of the invention. The following classes of compounds can be used for treating or suppressing the symptoms of conditions related to nervous system affliction, particularly addictive disorders. Examples of at least the following classes of compounds are provided for the method of the invention.

A suitable compound can have the formula, below: 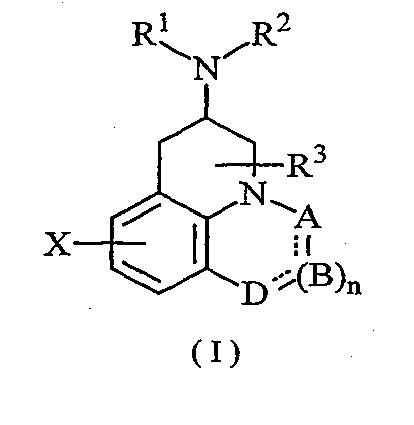 or a pharmaceutically acceptable salt thereof, wherein:
R¹, R², and R³ are each independently hydrogen, C₁₋₆ alkyl, C₃₋₅ alkenyl, C₃₋₅ alkynyl, C₃₋₇ cycloalkyl, C₄₋₁₀ cycloalkyl- or phenyl-substituted C₁₋₆ alkyl, or R¹ and R² are joined to form a C₃₋₇ cyclic amine which can contain additional heteroatoms and/or unsaturation;
n is 0 or 1;
X is hydrogen, C₁₋₆ alkyl, halogen, hydroxy, alkoxy, cyano, carboxamide, carboxyl, or carboalkoxyl;
A is CH, CH₂, CH-halogen, CHCH₃, C=O, C::S, C-SCH₃, C=NH, C-NH₂, C-NHCH₃, C-NHCOOCH₃, C-NHCN, SO₂, or N;
B is CH₂, CH, CH-halogen, C=O, N, NH, N-CH₃, or O; and
D is CH, CH₂, CH-halogen, C=O, O, N, NH, or N-CH₃.

Preferred compounds of the formula ( I ) are those wherein D is N or NH, n is 0, and R¹, R², R³, X, A, and B are as previously defined. Additional preferred compounds of formula ( I ) are those wherein A is CH, CH₂, CHCH₃, C=O, C=S, C-SCH₃, C=NH, C-NH₂, C-NHCH₃, C-NHCOOCH₃, or C-NHCN, and R¹, R², R³, n, X, B, and D are as previously defined.

More preferred compounds of formula ( I ) for the invention are those wherein A is CH or C=O, and R¹, R², R³, n, X, B, and D are as previously defined.

Compounds of formula ( I ) can be prepared by any suitable method. The compounds generally can be referred to as heterocyclic amine compounds. Methods for preparing compounds of formula ( I ) are further described in U.S. Patent No. 5,273,975, issued December 28, 1993, which is herein incorporated by reference.

Nonlimiting examples of formula ( I ) for the practice of the invention include, but are not limited to: 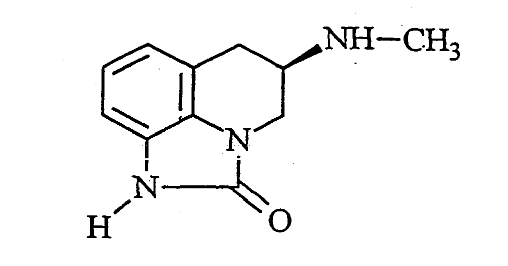 (R)-5,6-dihydro-5-(methylamino)-4H-imadazo[4,5,1-ij]-quinolin-2(1H)-one (uninverted CAS name) or (5R)-5-(methylamino)-5,6-dihydro-4H-imidao[4,5,1-ij]quinolin-(2H)-one (generated by ACD/Name software); 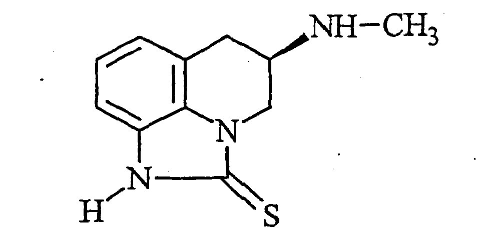 (5R)-5-(methylamino)-5,6-dihydro-4H-imidazo[4,5,1-ij]quinoline-2(1H)-thione; and pharmaceutically acceptable salts thereof.

Pharmaceutically acceptable salts include addition salts of both inorganic and organic acids. The pharmaceutically acceptable salts are preferred over the corresponding free amines since they produce compounds which are more water soluble and more crystalline. The preferred pharmaceutically acceptable salts include salts of the following acids hydrochloric, hydrobromic, sulfuric, phosphoric, nitric, citric, methanesulfonic CH₃-(CH₂)ₙ₁-COOH where n1 is 0 thru 4, HOOC-(CH₂)ₙ₁-COOH where n1 is as defined above, and HOOC-CH=CH-COOH. For other pharmaceutically acceptable salts, see *Int. J. Pharm*., 33, 201-217 (1986).

It is more preferred that the active agent (5R)-5-(methylamino)-5,6-dihydro-4H-imidazo[4,5,1-ij]quinoline-2(1H)-thione be present as the maleate salt, which is (5R)-5-(methylamino)-5,6-dihydro-4H-imidazo[4,5,1-ij]quinoline-2(1H)-thione maleate. A preferred salt of (5R)-5-(methylamino)-5,6-dihydro-4H-imidazo[4,5,1-ij]quinoline-2(1H)-thione is (5R)-5-(methylamino)-5,6-dihydro-4H-imidazo[4,5,1-ij]quinoline-2(1H)-thione 2-butenedioanate.

Other compounds suitable for the invention are those having the formula: 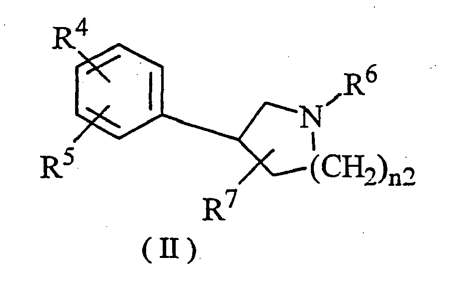 or a pharmaceutically acceptable salt thereof, wherein:
n2 is 0-3;
R⁴ and R⁵ are independently hydrogen, -OH, CN, CH₂CN, 2-CF₃, 4-CF₃, CH₂CF₃, CH₂CHF₂, CH=CF₂, (CH₂)₂CF₃, ethenyl, 2-propenyl, OSO₂CH₃, OSO₂CF₃, SSO₂CF₃, COR⁷, COOR⁷, CON(R⁷)₂, SOₓ₁CH₃, wherein x1 is 0-2, SOₓ₁CF₃, O(CH₂)ₓ₁CF₃, SO₂N(R⁷)₂, CH=NOR⁷, COCOOR⁷, COCOON(R⁷)₂, C₁₋₈ alkyl, C₃₋₈ cycloalkyl, CH₂OR⁷, CH₂(R⁷)₂, NR⁷SO₂CF₃, NO₂, halogen, a phenyl at positions 2, 3 or 4, thienyl, furyl, pyrrole, oxazole, thiazole, N-pyrroline, triazole, tetrazole or pyridine; provided that at least one of R⁴ and R⁵ is a substituent other than hydrogen and provided that when R⁴ or R⁵ is -OH R⁷ is other than hydrogen;
R⁶ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, -(CH₂)ₘ-R⁸, wherein m is 1-8, CH₂SCH₃ or a C₄-C₈ alkyl bonded to said nitrogen and one of its adjacent carbon atoms inclusive to form a heterocyclic structure;
R⁷ is independently hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, -(CH₂)ₘ-R⁸, wherein m is 1-8;
R⁸ is phenyl optionally substituted with a CN, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 2-thiophenyl, 3-thiophenyl, -NR⁹CONR⁹R¹⁰, or -CONR⁹R¹⁰; and
R⁹ and R¹⁰ are each independently hydrogen, C₁-C₈ alkyl, C₃-C₈ cycloalkyl, C₄-C₉ cycloalkylmethyl, C₂-C₈ alkenyl or C₂-C₈ alkynyl.

The preferred compounds are at least those compounds of formula ( II ) wherein:
R⁴ is CN, and n2, R⁵, R⁶, and R⁷ are as previously defined;
R⁵ is H, R⁶ is n-propyl, and n2, R⁴, and R⁷ are as previously defined;
R⁴ is -OSO₂CF₃, and n2 and R⁵-R⁷ are as previously defined;
R⁵ is H, R⁶ is C₁₋₈ alkyl, and n2, R⁴, and R⁷ are as previously defined;
R⁴ is 3-OH, R⁵ is H, R⁶ is n-propyl, R⁷ is a C₁₋₈ alkyl, and n2 is as previously defined;
n2 is 2, and R⁴-R⁷ are as previously defined; and
n2 is 0, and R⁴-R⁷ are as previously defined.

Compounds of formula ( II ) are described in U.S. Patent Nos. 5,594,024, issued January 14, 1997, and U.S. 5,462,947, issued October 31, 1995, each of which is incorporated herein by reference. The compounds can more generally be referred to as phenylazacycloalkane compounds.

Nonlimiting examples of formula ( II ) for the practice of the invention include, but are not limited to:
(3S)-3-[3-(methylsulfonyl)phenyl]-1-propylpiperidine hydrochloride;
(3S) -3- [3- (methylsulfonyl)phenyl] -1-propylpiperidine hydrobromide; and
(3S)-3-[3-methylsulfonyl)phenyl]-1-propylpiperidine (2E)-2-butenedioate.

More compounds suitable for the invention are the active agent cabergoline and derivatives thereof of the formula: 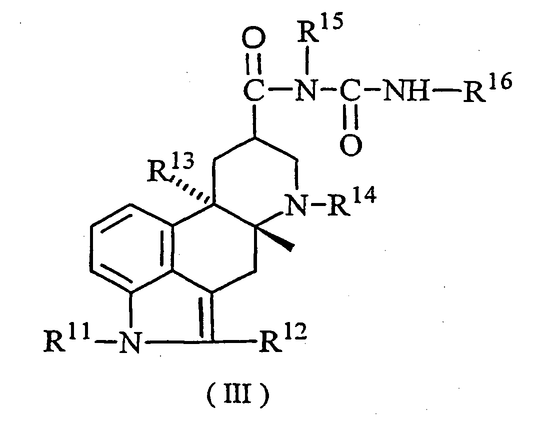 or a pharmaceutically acceptable salt thereof, wherein:
R¹¹ is hydrogen or methyl;
R¹² is independently hydrogen, halogen, methyl, formyl, S-R¹⁷, or SO-R¹⁷, wherein R¹⁷ is C₁-C₄ alkyl or phenyl;
R¹³ is hydrogen or methoxy;
R¹⁴ is independently C₁-C₄ alkyl, C₁-C₄ alkenyl, C₁-C₄ alkynyl, benzyl, or phenyl; and
R¹⁵ and R¹⁶ are each independently C₁-C₄ alkyl, cyclohexyl, phenyl optionally substituted with halogen or methoxy, or (CH₂)ₙ₃N(CH₃)₂, wherein n3 is an integer.

The chemical name for cabergoline is 1-((6-allylergolin-8β-yl)carbonyl)-1-(3-(dimethylamino)propyl)-3-ethylurea. Cabergoline is the generic name for the active ingredient in DOSTINEX™ (Pharmacia & UpJohn, Inc., Kalamazoo, Michigan, now Pharmacia Corporation), which is sold in the United States as a treatment for hyperprolactinemic disorders, and CABASER™ (Pharmacia & UpJohn, Inc.), which is sold in Europe as a treatment for Parkinson's disease. The synthesis and use of cabergoline and some useful derivatives thereof are disclosed and claimed in U.S. Patent No. 4,526,892, which is incorporated herein by reference. More specifically, the compounds disclosed generically and specifically in claims 1-4 of U.S. Patent No. 4,526,892 are incorporated herein by reference.

Another class of compounds suitable for the invention is the aromatic bicyclic amine compounds of the formula: 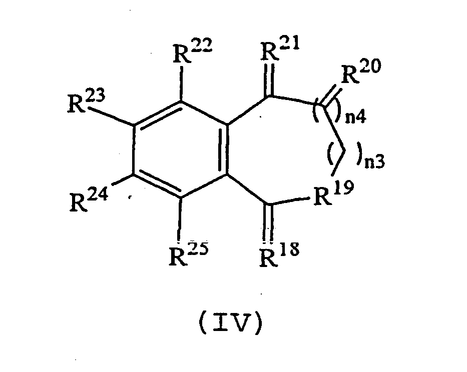 wherein:
n3 is 0 or 1;
n4 is 0 or 1, provided that R²⁰ is not present when n4 is 0;
R¹⁸ (1) is *α*-R¹⁸⁻¹:β-R¹⁸⁻² where one of R¹⁸⁻¹ or R¹⁸⁻² is selected from the group consisting of H or C₁-C₆ alkyl, and the other of R¹⁸⁻¹ or R¹⁸⁻² is a group of the formula : 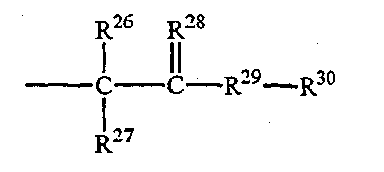
wherein R²⁶ and R²⁷ are independently selected from H or C₁-C₆-alkyl; R²⁸ is oxygen (O) or R²⁸ is *α*-R²⁸⁻¹:β-R²⁸⁻², wherein R²⁸⁻¹ and R²⁸⁻² are independently selected from H or C₁-C₆ alkyl R²⁹ is selected from the group consisting of: 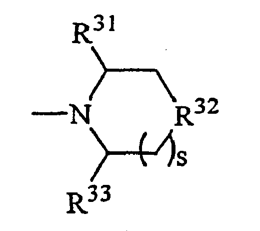 wherein R³¹ and R³³ are independently selected from H or C₁-C₆ alkyl; R³² is nitrogen (N-) or methine (HC-); and s is 1 or 2; 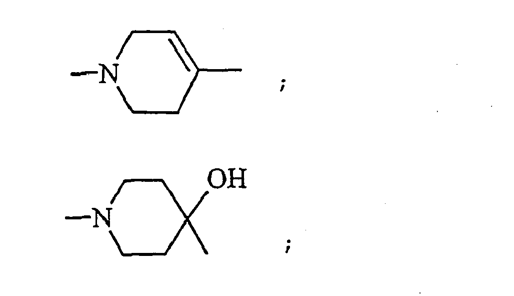 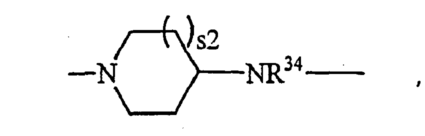 and
wherein R³⁴ is selected from the group consisting of H, C₁-C₆ alkyl, C₃-C₇ cycloalkyl, -C₁-C₃ alkyl-(C₃-C₇ cycloalkyl) ; and S2 is 0, 1, or 2; 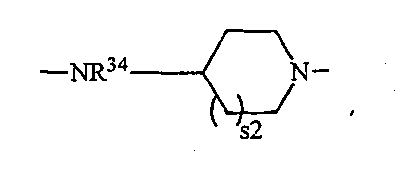 wherein R³⁴ and s2 are as defined above;
R¹⁹ is oxygen (O) or sulfur (S);
R²⁰ is *α*-R²⁰⁻¹: β-R²⁰⁻¹, wherein one of R²⁰⁻¹ and R²⁰⁻² is H, C₁-C₆ alkyl, and the other of R²⁰⁻¹ or R²⁰⁻² is H, C₁-C₆ alkyl, phenyl, hydroxy, and -O-(C₁-C₃ alkyl);
R²¹ is *α*-R²¹⁻¹: β-R²¹⁻¹, wherein one of R²¹⁻¹ and R²¹⁻² is H, C₁-C₆ alkyl, and the other of R²¹⁻¹ or R²¹⁻² is H, C₁-C₆ alkyl, phenyl, hydroxy, and -O-(C₁-C₃ alkyl);
and when n4 is 1, one of R²⁰⁻¹ or R²⁰⁻² and one of R²¹⁻¹ or R²¹⁻² can be taken together with the carbon atoms to which they are attached to form a carbon ring of 5-, 6-, or 7- members;
R²² is H, F, Cl, Br, I, -CONR³⁵R³⁶, -SONR³⁵R³⁶, CF₃, NR³⁵R³⁶, NO₂, CN, -NR³⁵-CO-R³⁶, -SO₂CF₃, C₁-C₄ alkyl, Si(CH₃)₃, and phenyl optionally substituted with one or two substituents selected from the group consisting of F, Cl, Br, I, and -CO-NR³⁵R³⁶, wherein R³⁵ and R³⁶ are independently selected from the group consisting of H, C₁₋C₆ alkyl, C₃-C₇ cycloalkyl, and -C₁-C₃ alkyl-(C₃-C₇ cycloalkyl);
and where R²² and one of R²¹⁻¹ or R²¹⁻² are taken together with the carbon atoms to which they are attached to form a carbon ring of 5-, 6-, or 7-members;
R²³ is H, F, Cl, Br, I, -CONR³⁷R³⁸, -SONR³⁷R³⁸, CF₃, NR³⁷R³⁸, NO₂, CN, -NR³⁷-CO-R³⁸, -SO₂CF₃, C₁-C₄ alkyl, Si(CH₃)₃, and phenyl optionally substituted with one or two substituents selected from the group consisting of F, Cl, Br, I, and -CO-NR³⁷R³⁸, wherein R³⁷ and R³⁸ are independently selected from the group consisting of H, C₁₋C₆ alkyl, C₃-C₇ cycloalkyl, and -C₁-C₃ alkyl- (C₃-C₇ cycloalkyl);
R²⁴ is H, F, Cl, Br, I, -CONR³⁹R⁴⁰, -SONR³⁹R⁴⁰, CF₃, NR³⁹R⁴⁰, NO₂, CN, -NR³⁹-CO-R⁴⁰, -SO₂CF₃, C₁-C₄ alkyl, Si(CH₃)₃, and phenyl optionally substituted with one or two substituents selected from the group consisting of F, Cl, Br, I, and -CO-NR³⁹R⁴⁰, wherein R³⁹ and R⁴⁰ are independently selected from the group consisting of H, C₁₋C₆ alkyl, C₃-C₇ cycloalkyl, and -C₁-C₃ alkyl- (C₃-C₇ cycloalkyl);
R²⁵ is H, F, Cl, Br, I, -CONR⁴¹R⁴², -SONR⁴¹R⁴², CF₃, NR⁴¹R⁴², NO₂, CN, -NR⁴¹-CO-R⁴², -SO₂CF₃, C₁-C₄ alkyl, Si(CH₃)₃, and phenyl optionally substituted with one or two substituents selected from the group consisting of F, Cl, Br, I, and -CO-NR⁴¹R⁴², wherein R⁴¹ and R⁴² are independently selected from the group consisting of H, C₁₋C₆ alkyl, C₃-C₇ cycloalkyl, and -C₁-C₃ alkyl-(C₃-C₇ cycloalkyl);
with the proviso that not more than two of R²², R²³, R²⁴, and R²⁵ are other than H; and
R³⁰ is selected from the group consisting of:
   phenyl optionally substituted with one or two substituents selected from the group consisting of CF₃, COR⁴³, COOR⁴³, CN, NO₂, NR⁴⁴-CO-R⁴⁵, -S-(C₁-C₆ alkyl), NR⁴⁴R⁴⁵, or a group represented by R⁴⁶;
   2-, 3-, and 4-pyridinyl optionally substituted with one or two substituents represented by R⁴⁶; and
   2-, 4-, and 5-pyrimidinyl optionally substituted with one or two substituents represented by R⁴⁶;
wherein R⁴³, R⁴⁴ and R⁴⁵ are independently selected from the group consisting of H, C₁-C₆ alkyl, C₃-C₇ cycloalkyl,
-C₁-C₃ alkyl-(C₃-C₇ cycloalkyl) ; and R⁴⁶ is selected from the group consisting of F, Cl, Br, I, -CO-NR⁴⁴R⁴⁵, - SO₂NR⁴⁴R⁴⁵, OH, SH, C₁-C₆ alkyl, C₃-C₆ cycloalkyl, -OR⁴⁷, - CH₂-(C₃-C₆ cycloalkyl), -CH₂-phenyl, C₃-C₆ cycloalkyl, SO₂CF₃, and
-CH₂CF₃, wherein R⁴⁴ and R⁴⁵ are as previously defined and R⁴⁷ is C₁-C₆ alkyl;
and enantiomers and diasteromers thereof, where such exist, and pharmaceutically acceptable salts thereof.

Compounds of formula (IV) are described in U.S. Patent No. 5,877,317, issued March 2, 1999, which is herein incorporated by reference. Aromatic bicyclic amine compounds, as well as methods for making and using the compounds, are disclosed in U.S. Patent No. 5,877,317. More particularly, aromatic bicyclic amine compounds are claimed in claims 1-18 of U.S. Patent No. 5,877,317.

Preferred compounds of formula (IV) are those wherein one of the substituents represented by R¹⁸⁻¹ or R¹⁸² is H, and the other substituent represented by R¹⁸⁻¹ or R¹⁸⁻² is a group of the formula: 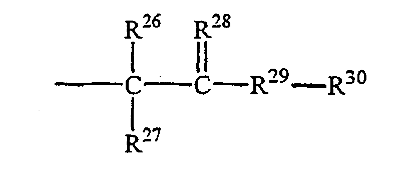 wherein R²⁶, R²⁷, R²⁸, R²⁹ and R³⁰ are as previously defined.

Nonlimiting examples of formula (IV) for the practice of the invention include, but are not limited to, compounds selected from the group consisting of:
1-(4-fluorophenyl)-4-[2-(isochroman-1-yl) ethyl] piperazine,
1-[2-(isochroman-1-yl)ethyl]-4-phenylpiperazine,
1-[2-(isochroman-1-yl)ethyl]-4-(4-methoxyphenyl) piperazine,
(-)-4-[4-[2-(isochroman-1-yl)ethyl]piperazin-1-yl]benzamide, and
(-)-4-[4-[2-(isochroman-1-yl)ethyl]piperazin-1-yl]benzenesulfonamide.

The preferred compound is (-)-4-[4-[2-(isochroman-1-yl) ethyl] piperazin-1-yl] benzenesulfonamide, or (-)-4-[4-[2-(3,4-dihydro-1H-2-benzopyran-1-yl)ethyl]-1-piperazinyl] -benzenesulfonamide, or 4-(4-(2-[(1S)-3,4-dihydro-1H-isochromen-1-yl]ethyl)-1-piperazinyl)benzenesulfonamide (Generated by ACD/Name software).

The term "alkyl" as used herein refers to The notation "C_{y}-C_{z}" denotes a hydrocarbon chain containing from y carbon atoms to z carbon atoms. For example, the term C₁-C₆ alkyl refers to a straight or branched alkyl group of from about 1 to about 6 carbon atoms, such as methyl, ethyl, n-propyl, iso-propyl, n-butyl, iso-butyl, sec-butyl, tert-butyl, n-pentyl, neopentyl, n-hexyl, iso-hexyl, and the like.

As used herein, the term "alkenyl" refers to a radical of an aliphatic, unsaturated hydrocarbon containing at least one double bond, including branched and unbranched forms. Examples of alkenyl groups include, but are not limited to, ethenyl, 1-methyl-1-ethenyl, 1-propenyl, 2-propenyl, 1-butenyl, 2-butenyl, 3-butenyl, 2-methyl-1-butenyl, 1-pentenyl, 2-pentenyl, 3-pentenyl, 4-pentenyl, 1-methyl-4-pentenyl, 3-methyl-1-pentenyl, 3-methyl,-2-pentenyl, 1-hexenyl, 2-hexenyl, 3-hexenyl, and the like.

The term "alkynyl" as used herein refers to an aliphatic unsaturated hydrocarbon containing at least one triple bond, including branched and unbranched forms. Examples of alkynyl groups are 1-propynyl, 2-propynyl, 1-butynyl, 2-butynyl, 3-butynyl, 2-methyl-1-butynyl, 1-pentynyl, 2-pentynyl, 3-pentynyl, 4-pentynyl, 1-methyl-4-pentynyl, 3-methyl-1-pentynyl, 3-methyl,-2-pentynyl, 1-hexynyl, 2-hexynyl, 3-hexynyl, and the like.

The term "cycloalkyl" as used herein refers to nonaromatic cyclic hydrocarbon group, preferably containing from three to six carbon atoms. Examples of cycloalkyl are cyclopropyl, cyclobutyl, cyclopentyl, cyclohexyl, and the like. Cycloalkyl groups also can have alkyl and alkoxy groups, as defined above, as well as halo substituents, for example, bromo, chloro, iodo, and fluoro.

The term "cycloalkyl-substituted alkyl" as used herein refers to an alkyl group as defined above, wherein at least one carbon atom of the alkyl group is attached to a cycloalkyl group as defined above.

As used herein, the term "phenyl-substituted alkyl" as used herein refers to an alkyl group as defined above, wherein at least one carbon atom of the alkyl group is attached to a phenyl group, i.e. a substituted or unsubstituted radical derivatized from benzene comprising a 6-membered aromatic ring.

The term "halogen" as used herein refers to the typical halogen atoms, for example, bromine, chlorine, iodine, and fluorine.

The term "hydroxy" refers to the group -OH.

The term "alkoxy" as used herein refers to a straight or branched hydrocarbon group as defined above attached to the parent molecule through an oxygen heteroatom, typically by a carbon to oxygen bond. The hydrocarbon of the alkoxy group preferably contains from 1 to 6 carbon atoms. Typical alkoxy groups are methoxy, ethoxy, n-propoxy, isopropoxy, n-butoxy, sec-butoxy (1-methylpropoxy), t-butoxy (1,1-dimethylethoxy), n-pentoxy, t-pentoxy (1,1-dimethylpropoxy), and the like.

The term "aryl" as used herein refers to an aromatic cyclic hydrocarbon, such as phenyl and naphthyl. The aryl group, such as phenyl groups, optionally can be substituted with alkyl, alkoxy or a halo group, for example, bromo, chloro, iodo, and fluoro. Examples of aryl groups include, but are not limited to, phenyl, bromophenyl, chlorophenyl, iodophenyl, fluorophenyl, bromonaphthyl, and the like.

The term "cyano" as used herein refers to the group -CN.

The term "carboxamide" as used herein refers to the group -CONH₂.

The term "carboxyl" as used herein refers to the group -COOH.

The term "carboalkoxyl" as used herein refers to a group -COOR wherein R is ower alkyl, such as carboxymethoxy, carboethoxy, and the like.

The term "thienyl" as used herein refers to the radical derived from thiophene.

The term "furyl" as used herein refers to the radical derived from furan, and its derivatives, including tetrahydrofuran, e.g. tetrahydrofuryl.

The term "pyrrole" as used herein refers to all isomers of the pyrrole ring, including 2H-pyrrole, pyrrole, 2-pyrroline, and like.

The term "cycloalkylmethyl" as used herein refers to a cycloalkyl group attached to the parent compound by a methylene (-CH₂-) group.

"Pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability. More particularly, the term "pharmaceutically acceptable salts" as used herein refers to organic and inorganic acid addition salts of the parent compound.

The dosages to be given with the compounds above can be easily determined by a skilled physician with experience in prescribing biologically active drugs designed to modulate central nervous system, movement and related psychological and physiological disorders, preferably of the disorders described herein. While the active agent generally is administered once a day or twice a day, it can be administered more, or less, frequently, as is suitable, and in the dosages desired for the particular patient.

Any conventional pharmaceutical preparations can be used, e.g., consisting essentially of an inert pharmaceutical carrier and an effective dose of the active substance; e.g., plain or coated tablets, capsules, lozenges, powders, solutions, suspensions, emulsions, syrups, suppositories, transdermal patch, and other useful mediums for delivering the active agent. Preferably, the active agent is formulated into oral dose tablets.

Preferred oral dose tablets comprise the active agent and a pharmaceutically acceptable carrier. The preferred pharmaceutically acceptable carrier can comprise one or more inert excipients, for example, mannitol, maize starch, colloidal silica, povidone, and magnesium stearate.

Tablets containing heterocyclic amine compounds, phenylazacycloalkane compounds, and cabergoline or cabergoline-type compounds typically incorporate, in mg/tablet, the following amounts of active agent: 0.125, 0.25, 0.5, 1.0, 1.25 and 1.5 mg. The preferred starting dose for the administration of these compounds is about 0.125 mg/day, provided to a patient three times per day (tid). The dose may be increased from the initial dosage to a higher amount with increases every five to seven days up to a maximum dose of 10 mg/day. A preferred higher total daily dosage is about 6 mg/day. A more preferred higher dosage is about 4.5 mg/day to 5 mg/day.

Dosages of the aromatic bicyclic amine compounds can be from about 5 mg of the aromatic bicyclic amine active agent to about 120 mg of the aromatic bicyclic amine active agent per day. Preferably, an aromatic bicyclic amine active agent is administered in an amount of about 20 mg/day to about 100 mg/day. More preferably, an aromatic bicyclic amine active agent is administered in an amount of about 40 mg/day to about 80 mg/day. The aromatic bicyclic amine compounds, like other compounds suitable for the invention, can be administered at an initial dose strength that is later increased to a suitable maximum daily dose.

For treating the addictive disorders described herein the drug may also be provided in chewable format, such as a chewing gum. The amount of active drug included in a chewable base may be half the dosage suggested above for the tablet, for example starting with about 0.075 mg of cabergoline per square of chewing gum being administered tid and followed with higher levels after the patient shows tolerance to the drug. Chewing gum dosages contemplated within the scope of the invention include at least 0.075, 0.10, 0.125, 0.150 mg/day, in addition to those mentioned for a tablet for heterocyclic amine compounds, phenylazacycloalkane compounds, and cabergoline or cabergoline-type compounds. Similarly, dosages contemplated for the aromatic bicyclic amine compounds include from about 2.5 mg/day to about 125 mg/day. One or two chewing gum squares can be provided to the patient up to three times a day, depending on the therapeutic need of the recipient.

Transdermal administration, such as with a skin patch application, and inhalation therapy, such as with an inhaler, also are foreseen where the patch or inhaler would deliver desired levels of the active agent to the patient. A transdermal patch containing the active agent also could be combined with a patch containing nicotine to eliminate a patient's craving for tobacco-containing products.

The drug first is typically administered to a patient at a low dosage to avoid possible nausea that may occur with higher starting doses. The dose is then titrated up to higher levels until a suitable therapeutic effect is achieved.

The effective dose range can be from 0.01 mg/day to about 10.0 mg/day per patient for a heterocyclic amine, phenylazacycloalkane, cabergoline, or cabergoline-type derivative. The preferred effective dose is an amount of the active agent between about 0.125 mg/day and about 6 mg/day. The more preferred effective dose is an amount of the active agent between about 0.375 mg/day to about 5 mg/day. An especially preferred effective dose is an amount of the active agent between about 0.75 mg/day and 4.5 mg/day to a patient. In addition to being administered by oral or intravenous route, the active agent also can be administered transdermally or by inhalation.

In the practice of the invention, typically a starting dose of about 0.125 mg/day, administered three times per day, is incrementally increased every five to seven days until optimal therapeutic effect is achieved. The dosage can be titrated to achieve a maximal therapeutic effect, provided that the patient does not experience intolerable side effects. One ordinarily skilled in art of providing medicine, such as a physician or pharmacist can determine the optimal dosage level after considering a patient's age, size, medical history, responsiveness to and toleration for the drug.

Addictive disorders and psychoactive substance use disorders, such as intoxication disorders, inhalation disorders, alcohol addiction, tobacco addiction and/or nicotine addiction can be treated according to the invention. Tobacco and nicotine addiction would be treated with the goal of achieving either smoking cessation or at least a reduction in the intake of tobacco and/or nicotine. General descriptions of addictive disorders, including disorders related to intoxication, inhalants, and tobacco addiction or nicotine addiction can be found in many standard sources. The addictions and behaviors that can be treated by the invention generally are further described in, for example, *The American Psychiatric Press Textbook of Psychiatry, Second Edition,* edited by Robert E. Hales, Stuart C. Yudofsky, and John A. Talbott, 1994, incorporated by reference, especially pp. 401 et. seq., section on "Nicotine" incorporated by reference; and *Manual of Psychia tri c Therapeutics, Second Edition,* edited by Richard I. Shader, incorporated by reference, especially pp. 85 from Chapter 11, entitled "Hypnosis".

The method is particularly useful for the treatment of
and relief from alcohol and other psychoactive substance use disorders such as, for example, disorders related to intoxication or inhalants, more particularly tobacco or nicotine addiction. The effect of the invention on tobacco addiction more particularly involves the administration of the active agent in a manner and form that reduces the symptoms of the disease. In particular, the tobacco- and/or nicotine-related aspect of the invention can be used to reduce or stop the smoking or chewing of nicotine-containing materials by a patient.

Without further elaboration, it is believed that one skilled in the art can, using the preceding description, practice the invention to its fullest extent. Those skilled in the art will promptly recognize appropriate variations from the procedures both as to reactants and as to reaction conditions and techniques.

## Claims

1. Use of a compound for the preparation of a medicament for treating or suppressing the symptoms of at least one disorder selected from addictive disorders, psychoactive substance use disorders, intoxication disorders, inhalation disorders, alcohol addiction, tobacco addiction, and nicotine addiction, wherein the compound is of the formula: 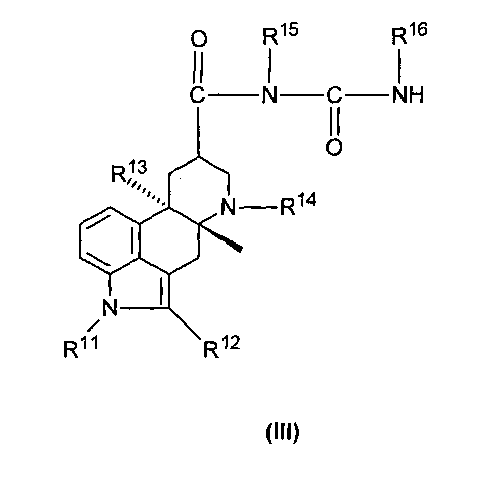 or a pharmaceutically acceptable salt thereof, wherein:
R¹¹ is hydrogen or methyl;
R¹² is hydrogen, halogen, methyl, formyl, S-R¹⁷, or SO-R¹⁷ wherein R¹⁷ is C₁-C₄ alkyl or phenyl;
R¹³ is hydrogen or methoxy;
R¹⁴ is C₁-C₄ alkyl, C₁-C₄ alkenyl, C₁-C₄ alkynyl, benzyl or phenyl; and
R¹⁵ and R¹⁶ are each independently C₁-C₄ alkyl, cyclohexyl, benzyl, phenyl optionally substituted with halogen or methoxy, or (CH₂)ₙN(CH₃)₂, wherein n is an integer.

2. The use of claim 1, wherein the compound is 1-((6-allylergolin-8β-yl)carbonyl)-1-(3-(dimethylamino)propyl)-3-ethylurea.

3. The use of claim 1 or claim 2, wherein the disorder is tobacco and/or nicotine addiction.

4. The use of claim 1 or claim 2, wherein the active agent is used to reduce the smoking or chewing of nicotine-containing materials.

5. The use of any preceding claim, wherein the compound is to be administered in a dose of 0.01 to 10.0 mg/day.

6. The use of claim 5, wherein the dose is 0.125 to 6 mg/day.

7. The use of claim 6, wherein the dose is 0.375 to 5 mg/day.

8. The use of claim 7, wherein the dose is 0.75 to 4.5 mg/day.

9. The use of any of claims 5 to 8, wherein the dose is about 0.125 mg/day to be administered three times per day and titrated to higher levels every five to seven days up to a maximum dose of 10 mg/day.
